# EUROPEAN PATENT SPECIFICATION

(11) **EP 0 300 806 B1**
(45) Date of publication and mention of the grant of the patent: **07.09.1994**
(21) Application number: 88306738.1
(22) Date of filing: 22.07.1988
(51) Int. Cl.: A61K 31/485, A61K 9/50

(54) **Opioid analgesic liposomal delivery-release system**
Liposom-Freisetzungssystem für Opoid-Analgetika
Système de libération liposomique d'opoides analgésiques

(30) Priority: 24.07.1987 US 77155
(43) Date of publication of application: 25.01.1989
(73) Proprietor: VESTAR, INC., San Dimas California 91773 (US)
(72) Inventor: Richer, Leroy Leonard, San Gabriel, CA 91775 (US)
(74) Representative: Goldin, Douglas Michael

(56) References cited:
- EP-A- 0 152 379
- FR-A- 2 592 791
- JOURNAL OF PHARMACEUTICAL SCIENCES, vol. 74, no. 9, September 1985, American Pharmaceutical Association, Washington, DC (US); A.L.WEINER et al., pp. 922-925.
- ACTA PHYSIOLOGICA ET PHARMACOLOGICA BULGARICA, vol. 12, no. 4, 1986, Sofia (BG); K.YAKIMOVA et al., pp. 12-18.

## Description

This invention relates to a novel opioid liposomal delivery and release system. In another aspect this invention relates to an improved method for increasing the therapeutic efficacy of the nalorphine-cyclazocine class of drugs. In another aspect, it relates to the administration of liposomal delivery and release vesicles for the improved therapeutic efficacy of butorphanol.

Included in the many recent advances made in the field of drug administration is development of controlled or sustained drug release systems. Controlled release systems do not represent efforts to target drugs to specific organs; rather, such systems constitute devices for the continuous release of drug into a particular compartment of the body, such as the blood, gastrointestinal tract, etc. One clear benefit of such systems is that they constitute an alternative to multiple dosage regimens. A variety of technologies exist for obtaining controlled drug release ranging from chemical and mechanical approaches (for example, osmotically driven pumps, erodible polymeric matrix devices) to biological approaches (for example, phospholipid vesicles, immunotoxins). See generally M. J. Poznansky and R. L. Juliano, Biological Approaches to the Controlled Delivery of Drugs: A Critical Review in 36 Pharmacological Review, 277-336 (1984).

Phospholipid vesicles or liposomes are a popular approach to controlled drug delivery and have been considered for the delivery of drugs by the parenteral route. See generally P. Speiser, Ultrafine Solid Compartments as Carriers for Drug Delivery in Optimization of Drug Delivery, 309-11 (1981). In that context, a single dose of liposomal AraC (cytosine arabinosine) has been shown to be effective in prolonging the lives of mice having leukemia, whereas single-dose treatment with free drug is totally ineffective -- a result attributed at least in part to the time-release action of liposomes. See studies cited in M. J. Poznansky and R. L. Juliano, supra at 302. Additionally, a single injection of liposomal formulations, incorporating either growth hormone or insulin, has been shown to provide prolonged release of the subject drug as compared to single injection doses of free drug. A. Weiner, S. Carpenter-Green, E. Soehngen, R. Lenk and M. Popescu, Liposome-Collagen Gel Matrix: A Novel Sustaining Drug Delivery System in 74 Journal of Pharmaceutical Sciences, 922-25 (1985).

It has also been found desirable to administer analgesics through sustained release vehicles especially where large doses must be given for prolonged periods of time. It has been found, for example, that tablets of enteric coated aspirin microspheres can be given half as frequently and in twice the dosages as free aspirin. Green, D.M. Tablets of Coated Aspirin Microspherules - A New Dosage Form, The Journal of New Drugs, Sept.-Oct., 294-304, 1966. It would be particularly desirable if sustained release technology could be used for analgesics which must be given parenterally.

Butorphanol, marketed as Stadol® (Bristol-Myers Laboratories, Inc.) in a parenteral formulation of a tartrate salt, is the subject of United States Patent No. 3,819,635. It is a synthetic opioid agonist-antagonist which is highly effective for the treatment of acute and chronic pain. Parenteral butorphanol is several times as potent as morphine and most other popular morphine analogues; yet, studies indicate that the chronic administration of butorphanol followed by deprivation does not lead to compulsive drug-seeking behavior in humans. See studies cited in I.J. Pachter and R. Evens, Butorphanol in 4 Drug and Alcohol Dependence 329-31 (1985). But see S. I. Smith and W. M. Davis, Nonmedical Use of Butorphanol and Diphenhydramine in 252 JAMA 1010 (1984).

Butorphanol is metabolized in the liver with renal excretion. The elimination of butorphanol and its metabolites is rapid and the duration of analgesia is usually in the range of 3 to 4 h, with maximal analgesia obtained at 0.5 to 1 h.

Parenteral butorphanol is at present most attractive for use in the treatment of acute pain associated with surgery. In this application, butorphanol is given preoperatively and perioperatively as needed to maintain efficacy.

Butorphanol is also used for the treatment of severe postoperative pain and for some conditions of chronic pain. Specifically, the drug has proven effective in the treatment of pain associated with gastrointestinal, gynecological, obstetrical and orthopedic procedures as well as pain secondary to burns and pain caused by renal calculi. Additionally, chronic pain secondary to cancer, neurologic/spastic disease, and musculoskeletal disorders can be reduced significantly by parenteral butorphanol. See studies cited in Pachter and Evens, supra at 332-33.

The standard dose of butorphanol tartrate is 2 mg, given by the intramuscular route for post operative pain. Doses up to 4 mg can be used for more persistent severe pain. The typical dosing interval ranges from 3 to 4 h. to sustain analgesia. Although the treatment of chronic pain by the parenteral route is extremely inconvenient, doses of 1 to 8 mg have been used at 6 h. intervals for periods as long as several months and occasionally years. Pachter and Evens, supra at 336-37. Higher doses do not extend the duration of analgesia. Physician's Brochure, Stadol®. Apart from the fact that multiple dosing is inconvenient, multiple dosing has been shown to produce tolerance (tachyphylaxis) to the analgesic effect of butorphanol. H. Blumberg, H.B. Dayton and P.S. Wolf, 123 Proceedings of the Society of Experimental Biology, 755 (1966).

It would be advantageous to provide a delivery system for controlled release of an analgesic as potent as butorphanol which would extend the duration of analgesia provided by a single injected dose without resulting in tachyphylaxis. Such a system would enhance the attractiveness of the nalorphine-cyclazocine drugs such as butorphanol in their current applications and, perhaps, would increase the number of conditions for which they would be beneficial.

### Summary of the Invention

The present invention provides the use of phospholipid vesicles encapsulating an opioid analgesic for the manufacture of a medicament for the treatment of pain in an injected dose, said vesicles containing an amount of opioid analgesic which is sufficient to extend the duration of analgesia beyond that obtainable by the injection of the same amount of analgesic when injected as free drug in a single dose but which produces essentially no tachyphylaxis when compared to that observed when the same amount of the opioid analgesic is administered in free drug form in at least 4 doses over the time period during which the vesicles provide analgesia. Injectable compositions for the controlled release of butorphanol comprising butorphanol tartrate encapsulated in liposomes consisting of phospholipids and cholesterol are described. Although only butorphanol is described, it is to be understood that the present invention is equally applicable to other members of the 14-hydroxymorphinan group such as morphine, meperidine, naloxone, and pentazocine, as well as other members shown to have a similar chemical reactivity and mechanism of action. These compositions extend the single injected dose duration of analgesia but produce essentially no tachyphylaxis. They are administered by the parenteral route to treat acute pain associated with surgery as well as postoperative pain and some conditions of chronic pain.

### Detailed Description of the Invention

Liposomal drug delivery vesicles can be designed and formulated to best fit any particular desired purpose. The most common rationale employed in liposomal design can be found in M.J. Poznansley and R.L. Juliano, Biological Approaches to the Controlled Delivery of Drugs: A Critical Review in 36 Pharmacological Review, 277-336 (1984). The Examples which follow illustrate the preparation, characterization and in vivo application in an animal model of encapsulated butorphanol tartrate using the compositions of the present invention.

The following examples are presented to illustrate the invention, and are not intended to limit the scope thereof.

### EXAMPLE I

### Vesicle-Butorphanol Tartrate Formulation

Lipid films of 40 mg distearoylphosphatidylcholine and cholesterol (mole ratio 2:1) were hydrated with 2 ml of 50 mg/ml butorphanol tartrate in phosphate buffered saline (PBS). Hydration times were varied from 2 h to 24 h. No advantage was observed for the longer hydration times. The aqueous suspension was briefly pulse sonicated to produce a more homogeneous mixture and then vortex stirred for 20 mins. The multilamellar vesicle (MLV) preparation was washed three times by centrifugation and resuspension in PBS. Prior to use in studies an aliquot of each MLV preparation was analyzed for butorphanol content.

### Characterization of Vesicle-Butorphanol Tartrate Formulation

The concentration of butorphanol in vesicle suspensions and blood samples was determined using a modified version of a high pressure liquid chromatography (HPLC) procedure provided by Bristol Laboratories. F.R. Sisto, Bristol Laboratories, Method No. 0330, version 003 (1983). The analysis was performed with Waters, Model 150 pumps, Model 710B WISP, and an ESA, Model 5100A Coulochem detector. The column used was a 25 cm x 4.6 mm Vydac diphenyl.

The mobile phase consisted of ammonium acetate and acetonitrile at concentrations and pH specified in the Bristol protocol. Prior to use the mobile phase was passed through a Millipore, Durapore membrane and degassed in vacuo. Column flow rate was 2 ml/min and eluant detection was accomplished using electrochemical detection (coulometric) with oxidative mode at + 1.2 V and guard cell at + 1.0 V. Standard solutions of butorphanol tartrate were prepared in pH 7 ammonium acetate. The standard solution was diluted and used directly as an external standard for HPLC. The same solution was also used to spike whole heparinized blood as a sample standard. In this case, after spiking with butorphanol, the blood was centrifuged and plasma butorphanol absorbed on a C-18 Bond-Elut column. Samples were eluted with pH 4.1 acetate buffer, 25%, and acetonitrile, 75%, and used directly for HPLC analysis.

### Extended Analgesia Studies

Duration of analgesia was evaluated using the phenylquinone-induced writhing assay essentially as described by Pircio et al. R.W. Pircio, J.A. Gylys, R.L. Cavanagh, J.P. Buyniski and M.E. Bierwagen, 220 Archives Internationales de Pharmacodynamie et de Therapie, 231-57 (1976). Mice were given intraperitoneal injections of 0.025% (w/v) phenyl-p-quinone in 5% ethanol (2.5 mg/kg). Beginning 5 minutes after the phenylquinone injection, the animals were observed for writhing and the number of writhes over a 10 min period was determined. The writhing phenomenon has been described by Siegmund et al. E. Siegmund, R. Cadmus, G. Lu, 95 Proceedings of the Society for Experimental Biology and Medicine, 729-31 (1957). Five animals per observer was established as a manageable procedure yielding values which were reproducible and consistent from observer to observer. Control animals exhibited a mean of 20 writhes per mouse. This is in agreement with the mean reported by Pircio, et al. For all studies reported here a total of 10 animals were used per datum.

Prior to initiating treatments requiring intramuscular injections of free and vesicle encapsulated butorphanol tartrate conditions for administering i.m. injections to mice were established and validated. Trial mice were given i.m. injections in the hind leg of various volumes of Trypan-blue stain (GIBCO Laboratories). A 27 gauge, 1/2" needle was used for the injections. The injected mice were then sacrificed and the injection site carefully dissected. When injection volumes below 200 µl were used, it was demonstrated that mice could be reproducibly injected in their muscle bundles. No traces of stain were evident subcutaneous nor along the bone. All i.m. injections mentioned in this report were adjusted to 50 µl injection volume.

The dose required for single injection ED₅₀ and ED₁₀₀ of free butorphanol tartrate was determined by administering various doses of free drug intramuscularly 15 minutes prior to the i.p. injections of phenylquinone. The dose which produced a 50% reduction in the mean number of writhes, as compared to the control group, was called the ED₅₀. The ED₅₀ and ED₁₀₀ values determined in this study were compared for agreement with reported literature values and used along with control values as a reference standard for each study. See Y. Lambert, J.P. Davis, I. Monkovic, Analgesics and Narcotic Antagonists in the Benzomorphan and S-Oxamorphinam Series, 5, 21 Journal of Medicinal Chemistry, 423-27 (1978); I. Monkovic, H. Wong, R.W. Pircio, Y. Perron, I. Pachler, B. Belleau, Oxilorphan and Butorphanol. Potent Narcotic Antagonists and Nonaddicting Analgesics in the 3, 14-Dihydroxymorphinan Senies: Part V. 53 Canadian Journal of Chemistry, 3094-102 (1975).

Extended analgesia studies were conducted using no fewer than 50 animals per treatment group. For a given treatment, animals were divided into subgroups of 10. Subgroups were used only once in efficacy measurements using the phenylquinone bioassay. The times at which analgesic effect was measured were based on the particular treatment and on the results of the previous bioassays for the treatment group.

### Results

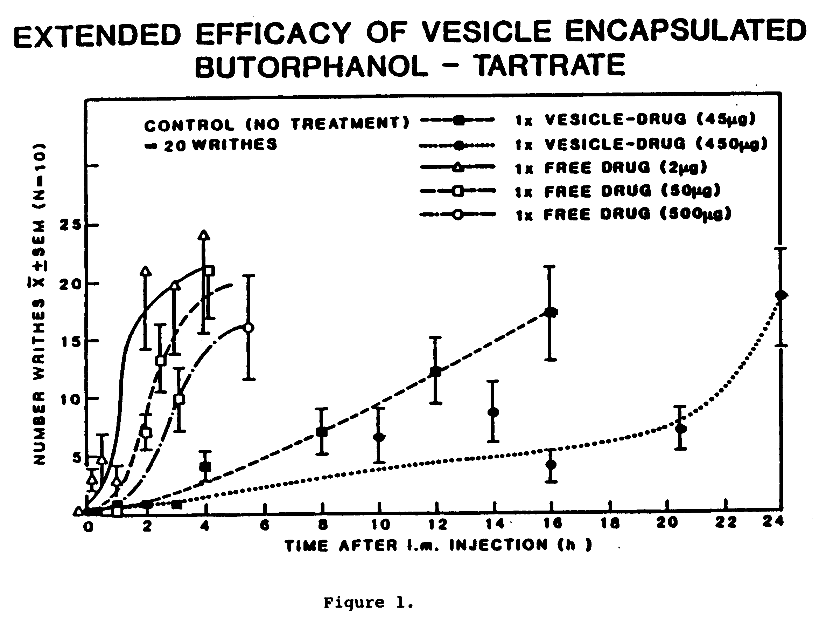

Shown in Fig. 1 are the extended analgesic effects achieved when a relatively low dose of vesicle encapsulated butorphannol tartrate is used for injection and those obtained when a higher dose is used, and the duration of analgesia for free butorphanol tartrate given as the ED₁₀₀ dose and at doses equivalent to the amounts administered in the two vesicle treatments.

The dose response curves for the three levels of free butorphanol tartrate agree with literature which indicates that higher doses do not extend the duration of analgesia. Physician's Brochure, Stadol®. The curves in Fig. 1 are visually drawn and the 500 ug dose curve has only two time points, zero and 5.5 h. However, based on the 2 ug and 50 ug dose curves it would appear that injections of higher doses of the free drug do not provide extended efficacy.

By contrast, the higher dose of vesicle-butorphanol tartrate did extend the duration of analgesia. Animals receiving the higher dose of vesicle encapsulated butorphanol tartrate did not exhibit the same number of writhes as controls until 24 h after treatment.

### EXAMPLE II

The vesicle-butorphanol tartrate formulation and the technique used to characterize that formulation are as described in Example I.

### Tolerance Studies: Tachyphylaxis with Free versus Vesicle Encapsulated Butorphanol Tartrate

The potential development of tolerance from vesicle-encapsulated butorphanol tartrate was examined in two different protocols. The intent in both protocols was to compare the magnitude of any shift in ED₅₀ dose, tolerance, resulting from the controlled release of butorphanol from vesicle encapsulated butorphanol tartrate with the degree of tolerance developed from multiple injections of free butorphanol tartrate, given on a schedule so as to produce approximately equivalent analgesia as the vesicle treatment.

In the first protocol 3 treatment groups were evaluated. The first group was a control, and received four intramuscular injections of 50 µl saline at 3 h intervals. The second group was given 4 i.m. injections of the single injection ED₁₀₀ dose of butorphanol tartrate (47 ug/kg) at 3 h intervals. The third group received a single i.m. injection of vesicle encapsulated butorphanol tartrate. The dose of vesicle-butorphanol tartrate had been previously shown to produce effective analgesia for at least 12 h. At least 50 mice were used per treatment group. Treatment groups were divided into subgroups of 10 mice, and subgroups were started on their prescribed treatment at 1 h. intervals. This afforded sufficient time for the ED₅₀ determinations which were run at the end of each treatment period.

Four hours after the last multiple injection treatment, or 13 h after the start of each treatment, the ED₅₀ for free i.m. butorphanol tartrate was again established for each treatment group. The same procedure was followed as described above.

The second protocol was designed after review of the results of the first protocol. From the results of the first protocol concern was raised that the animals which received vesicle encapsulated butorphanol tartrate were continuing to benefit from controlled release of butorphanol at the 13 h time point when the tolerance studies were run. Therefore, a study was designed to test for tachyphylaxis at a time when blood levels of butorphanol had returned to baseline.

In the second protocol two treatment groups were used. The first group received five i.m. injections of ED₁₀₀ free butorphanol tartrate at 3 h intervals. The second group received a single i.m. injection of the 12 h efficacy dose of vesicle encapsulated butorphanol tartrate. For both groups the ED₅₀ for free butorphanol tartrate was determined 36 h after the start of treatment.

Prior to executing the above study a pilot study was run to assure that butorphanol blood levels were at baseline values at 36 h. Animals were given the two treatments described above, sacrificed at 36 h and their blood collected and analyzed for butorphanol levels by HPLC. This pilot study served to demonstrate that the vesicle-butorphanol tartrate group was no longer receiving controlled release butorphanol at the time of the tolerance study.

### Results

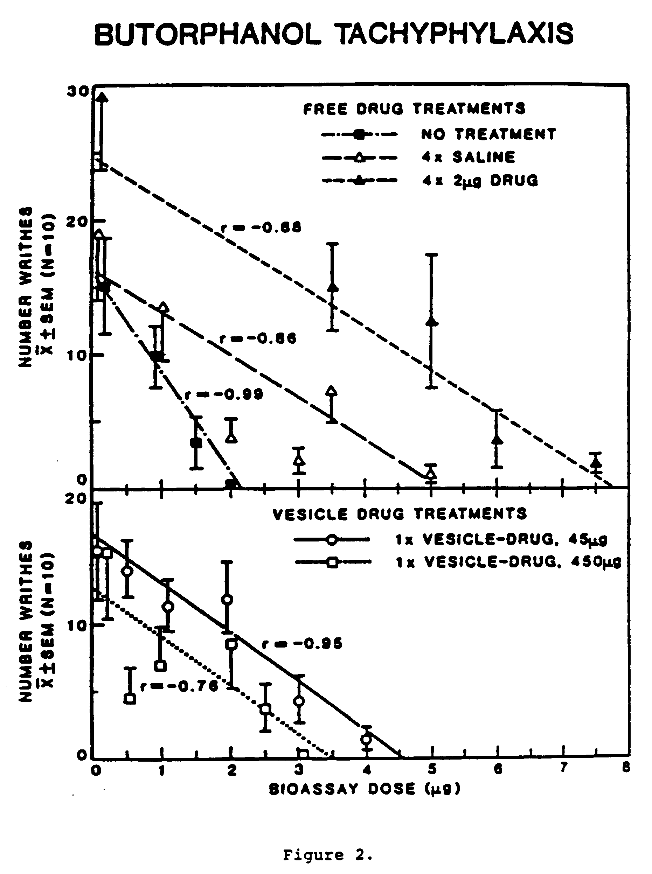

The results of the tolerance studies performed according to the two protocols are presented in a series of representations beginning with Fig. 2.

Fig. 2 presents the results of the tachyphylaxis studies run according to the first protocol. The plots are linear regression lines with means and standard errors of the means for the doses administered. In the top panel the regression line depicted by the solid squares is the dose response observed for a single injection of free butorphanol tartrate. These results were used to determine the ED₅₀ and ED₁₀₀ doses used in the remainder of the studies.

The line represented by closed triangles is the shift in the dose response to free butorphanol tartrate observed when animals have been previously treated with 4 injections of the ED₁₀₀ dose of free butorphanol tartrate. The magnitude of this shift in dose response, a 4-5 fold increase in ED₅₀ values between the no treatment animals (solid squares) and the multiple injection animals (solid triangles), was to serve as a reference in evaluating any tachyphylaxis observed in animals treated with vesicle-encapsulated butorphanol tartrate.

The open triangles in the top panel of Fig. 2 indicate the dose response observed when animals received 4 injections of physiological saline solution prior to bioassay with free butorphanol tartrate. This control exhibited a slight shift in ED₅₀ dose. This shift suggests that prior treatment of animals with substances that do not interact with opioid receptors will, nevertheless, produce tachyphylaxis.

The lower panel of Fig. 2 shows the results obtained with animals receiving vesicle encapsulated butorphanol tartrate. Thirteen hours after treatment with a single i.m. injection of vesicle-encapsulated butorphanol tartrate, at a dose shown to provide therapeutic efficacy for 12 h, the dose response to free butorphanol tartrate (open circles) was undistinguishable from the response observed with the multiple saline injections.

Also shown in Fig. 2 is a 13 h dose response for animals treated with a single injection of vesicle-butorphanol tartrate at a dose shown to be therapeutically effective for longer than 20 h. Since these animals were known to be benefitting from continued release of butorphanol they do not address the tachyphylaxis question and are included here for completeness.

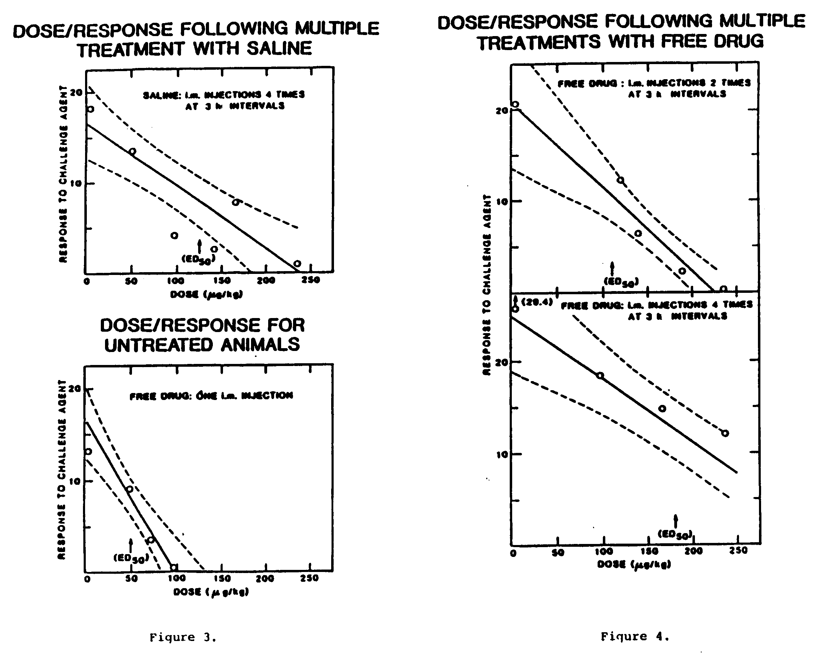

The data presented in a preliminary and consolidated form in Fig. 2 are presented in a different fashion in Figs. 3, 4 and 5. The plots in Fig. 3-5 are linear regression fits of the bioassay results expressed on a dose per body weight basis. Also shown are 95% confidence intervals about the regression lines.

The upper panel in Fig. 3 presents the results for control saline treatment. The ED₅₀ for free butorphanol tartrate, determined after the multiple injections of saline, shifted to the vicinity of 125 ug/kg. This is more than double the ED₅₀ determined in untreated mice (Fig. 3, bottom panel). The ED₅₀ in untreated animals was determined to be 50 ug/kg, which agrees with reported values. See Lambert, et al., supra; Monkovic, et al., supra.

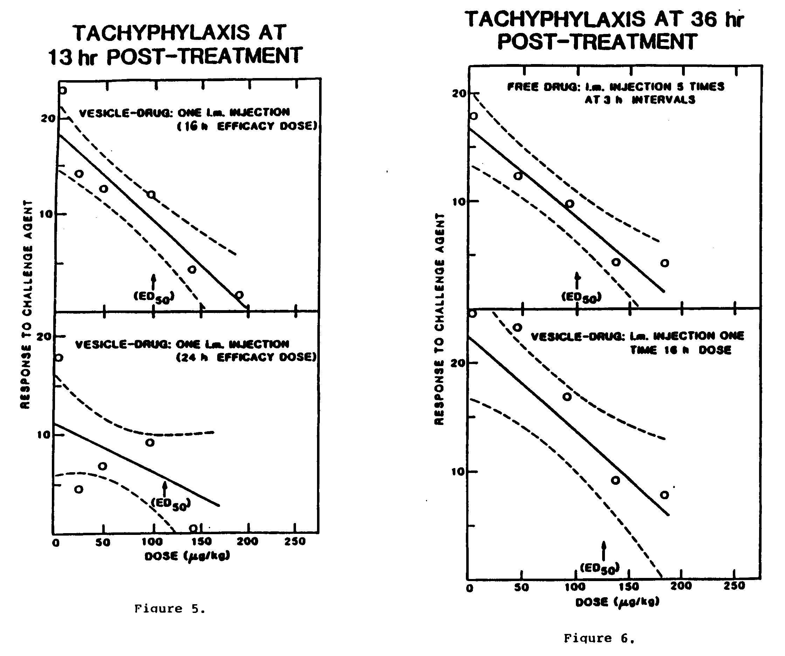

Multiple injections of free butorphanol tartrate produced the greatest tachyphylaxis in these series of studies. Treatment with two injections of 100 ug/kg butorphanol tartrate (the single injection ED₁₀₀ dose) shifted the ED₅₀ dose determined 4 h after the last injection to a value similar to that observed for the saline controls (Fig. 4, upper panel). Pretreatment with 4 injections of 100 ug/kg butorphanol tartrate shifted the ED₅₀ to approximately 4 times its initial value and nearly twice that observed in the saline-treated group (Fig. 4, bottom panel).

The ED₅₀ values, determined 13 h. after the start of treatment, for both doses of vesicle-butorphanol tartrate was equivalent to the value measured for saline controls (Fig. 5). The ED₅₀ values are not much different for the low and high doses of vesicle-butorphanol tartrate. Since it was shown that the high dose vesicle-butorphanol tartrate was therapeutically effective at the 13 h bioassay time, the ED₅₀ determined at this time was most likely erroneous.

Concern was raised that the low dose vesicle-butorphanol tartrate might also be releasing some butorphanol at the 13 h time point. If so, then the ED₅₀ dose determined in the bioassay would be incorrect.

A method for checking this has been described above as the second protocol run for tachyphylaxis. Basically, the second protocol extended the time for bioassay and redetermination of ED₅₀ values to 36 h after the start of treatment. For the case of vesicle encapsulated butorphanol tartrate treated animals a group of treated animals were bled at the 36 h time and individual samples analyzed for butorphanol. With a detection limit of 50 pg/ml HPLC analysis revealed measurable levels of butorphanol tartrate in the blood of 7 of 20 animals treated as described above. Five of the animals had blood butorphanol levels in the range 13-20 ng/ml; the remaining two animals levels of 469 and 344 ng/ml.

The results of the tachyphylaxis study run 36 h after the start of treatments are presented in Fig. 6. The group which received 5 injections of free butorphanol tartrate, 100 ug/kg, exhibited a 36 h ED₅₀ suggestive of recovery from tachyphylaxis (Fig. 6, top panel). This value was approximately the same as the ED₅₀ for vesicle-butorphanol tartrate (Fig. 6, bottom panel).

However, none of the groups treated with vesicle-butorphanol tartrate exhibited appreciable tolerance in these studies. The reason for this is not clear, and is certainly contrary to expectations of how a receptor-mediated process would behave in response to controlled therapeutic release.

## Claims

1. The use of phospholipid vesicles encapsulating an opioid analgesic for the manufacture of a medicament for the treatment of pain in an injected dose, said vesicles containing an amount of opioid analgesic which is sufficient to extend the duration of analgesia beyond that obtainable by the injection of the same amount of analgesic, when injected as a free drug in a single dose, but which produces essentially no tachyphylaxis in multiple dosing.

2. The use according to claim 1 in which the opioid analgesic is a member of the 14-hydroxymorphinan group.

3. The use according to claim 1 in which the opioid analgesic is selected from the group consisting of butorphanol, morphine and pentazocine.

4. The use according to any one of claims 1 to 3 in which the phospholipid vesicles are multilamellar.

5. The use according to any one of the preceding claims wherein the phospholipid vesicle includes a phospholipid and cholesterol.

6. The use according to any one of claims 1 to 3 wherein the phospholipid vesicle includes distearoylphosphatidylcholine and cholesterol in a mole ratio of 2:1.

## Patentansprüche

1. Verwendung von Phospholipidvesikeln, die ein opioides Analgetikum zur Herstellung eines Medikamentes zur Schmerzbehandlung in einer injizierten Dosis einkapseln, besagte Vesikel enthalten eine Menge eines opioiden Analgetikums, die ausreichend ist, die Dauer der Analgesie über das Maß zu verlängern, das durch dieselbe Menge eines Analgetikums erzielt wird, wenn es als eine freie Droge in einer einzelnen Dosierung injiziert wird, die aber im wesentlichen keine Tachyphylaxie in multiplen Dosierungen erzeugt.

2. Verwendung nach Anspruch 1, bei der das opioide Analgetikum ein Mitglied der 14-Hydroxymorphinangruppe ist.

3. Verwendung nach Anspruch 1, bei der das opioide Analgetikum aus der Gruppe, bestehend aus Butorphanol, Morphin und Pentazocin, ausgewählt ist.

4. Verwendung nach irgendeinem der Ansprüche 1 bis 3, bei der die Phospholipidvesikel multilamellar sind.

5. Verwendung nach irgendeinem der vorhergehenden Ansprüche, bei der das Phospholipidvesikel ein Phospholipid und Cholesterin einschließt.

6. Verwendung nach irgendeinem der Ansprüche 1 bis 3, bei der das Phospholipidvesikel Distearoylphosphatidylcholin und Cholesterin in einem molaren Verhältnis von 2:1 einschließt.

## Revendications

1. Utilisation de vésicules phospholipidiques encapsulant un opioïde analgésique pour la préparation d'un médicament pour le traitement de la douleur, sous forme de dose injectable, lesdites vésicules contenant une quantité d'opioïdes analgésiques qui est suffisante pour étendre la durée de l'analgésie au-delà de celle pouvant être obtenue par injection de la même quantité d'analgésique, quand injecté sous forme de principe actif libre en une dose unique, mais qui ne produit essentiellement pas de tachyphylaxie à doses multiples.

2. Utilisation selon la revendication 1 dans laquelle l'analgésique opioïde est un membre du groupe des 14-hydroxymorphinanes.

3. Utilisation selon la revendication 1, dans laquelle l'analgésique opioïde est choisi dans le groupe constitué par le butorphanol, la morphine et la pentazocine.

4. Utilisation selon l'une quelconque des revendications 1 à 3, dans laquelle les vésicules phospholipidiques sont multi-lamellaires.

5. Utilisation selon l'une quelconque des revendications précédentes dans laquelle la vésicule phospholipidique comprend un phospholipide et du cholestérol.

6. Utilisation selon l'une quelconque des revendications 1 à 3 dans laquelle la vésicule phospholipidique comprend de la distéaroylphosphatidylcholine et du cholestérol dans un rapport molaire de 2/1.
